# EUROPEAN PATENT APPLICATION

(11) **EP 4 659 751 A1**
(43) Date of publication of application: **10.12.2025**
(21) Application number: 24179973.3
(22) Date of filing: 04.06.2024
(51) Int. Cl.: A61K 31/7004, A61P 17/02

(54) **GALACTOSE AND FUCOSE MONOSACCHARIDES FOR RE-EPITHELIALIZATION AND TREATING LESIONS**

(71) Applicant: PathoBlock Therapeutics GmbH & Co. KG, 10117 Berlin (DE)
(72) Inventor: SCHUMACHER, Udo, 22085 Hamburg (DE); HÄNSCH, Inken Lena, 22085 Hamburg (DE); SCHUMACHER, Martin, 22085 Hamburg (DE)
(74) Representative: Richardt Patentanwälte PartG mbB

(57) **Abstract**

Disclosed is a composition (204) comprising galactose and fucose in monosaccharide form for use as a promotor of tissue re-epithelialization.

## Description

### FIELD OF THE INVENTION

The invention relates to compositions for treating lesions and/or promoting growth of tissue.

### BACKGROUND

The healing of wounds and treatment of lesions are critical aspects of medical care, affecting millions of people worldwide. Re-epithelialization, a pivotal phase in the wound healing process, involves the proliferation, migration and differentiation of epithelial cells to restore the integrity of the skin or mucosal barriers after an injury. Efficient and effective strategies to promote re-epithelialization and to treat lesions are of paramount importance for reducing the risk of infection, minimizing scarring, and ensuring a swift recovery for patients.

Traditional approaches to wound and/or lesion care have included the use of antiseptics, dressings, and, more recently, advanced therapies involving growth factors, cytokines, and cellular products. Despite these advancements, the quest for novel treatments that accelerate wound healing, enhance re-epithelialization, and minimize adverse outcomes remains ongoing.

In the state of the art, only compositions comprising galactose and fucose in polysaccharide form are known for treating wounds and/or lesions and/or for re-epithelialization of tissue.

US20170224602A1 describes a ferment extract and exopolysaccharide of a bacterial strain for its use in treatment and/or care of the skin, as well as its cosmetic and/or dermopharmaceutical compositions. In particular, its use for skin aging, skin firmness and hydration are described. It is not disclosed that the exopolysaccharide is broken down to monosaccharides e.g., by enzymes.

CN113307988A describes a preparation method of a galactofucosan sulfate hydrogel and application of the galactofucosan sulfate hydrogel in wound repair, in particular to application of the galactofucosan sulfate hydrogel in chronic wound repair. It is not disclosed that the galactofucosan sulfate is broken down to the individual monosaccharides e.g., by enzymes.

KR20030091582A describes a method for extracting polysaccharide from Bletilla striata Reichb. fil., the use of the extracted polysaccharide for moisturization, injury curing and cell proliferation, and a cosmetic composition containing the extracted polysaccharides. The polysaccharide can be formulated in the form of a cream or an emulsion. It is not disclosed that the polysaccharide is broken down to monosaccharides e.g., by enzymes.

Liu, Y.; Wu, N.; Geng, L.; Yue, Y.; Zhang, Q.; Wang, J. Fabrication of Sulfated Heterosaccharide/Poly (Vinyl Alcohol) Hydrogel Nanocomposite for Application as a Wound Healing Dressing. Molecules 2022, 27, 1801. https://doi.org/10.3390/molecules27061801 describes a hydrogel nanocomposite with poly(vinyl alcohol) (PVA) and sulfated heterosaccharide (UF). It is not disclosed that the UF is broken down to monosaccharides e.g., by enzymes. Furthermore, in light of the publication, it may be desirable to keep the viscous properties of the sulfated heterosaccharide in order to obtain a hydrogel, wherein breaking down of the sulfated heterosaccharide into monosaccharides may destroy its viscous properties.

Kössi J, Peltonen J, Ekfors T, Niinikoski J, Laato M. Effects of hexose sugars: glucose, fructose, galactose and mannose on wound healing in the rat. Eur Surg Res. 1999;31(1):74-82. doi: 10.1159/000008623, PMID: 10072613, describes the effects of four hexose sugars (D-glucose, D-fructose, D-galactose, D-mannose) on the developing granulation tissue in rats, and the findings of the study demonstrate that galactose may enhance wound healing in particular of granulation tissue, which comprises fibroblasts.

### SUMMARY OF THE INVENTION

It is an objective to provide an improved composition for use as a promotor of tissue re-epithelialization and/or for use in treating lesions. The objectives underlying the invention are solved by the features of the independent claims.

In an aspect, a composition comprising galactose and fucose in monosaccharide form for use as a promotor of tissue re-epithelialization is disclosed.

For example, the tissue is epithelial tissue. For example, the tissue is epithelial tissue of a skin. For example, epithelial tissue of a skin comprises epidermal cells. For example, the tissue is epidermal tissue. For example, the use as a promotor of tissue re-epithelialization comprises a use as a medicament and/or drug and/or agent defined by its function as a promotor of tissue re-epithelialization. For example, the tissue is tissue of a vertebrate, in particular of a mammal, in particular of a human. For example, the tissue is fibroblast-free tissue. For example, the tissue is an epidermis, an epithelial tissue in the central cornea, an engineered tissue. For example, the tissue may be a fibroblast-free tissue. A fibroblast-free tissue as used herein may be a tissue comprising less than 1% fibroblasts, in particular less than 0.1% fibroblast, in particular comprises 0% fibroblasts. In comparison to granulation tissue, which comprises fibroblasts, epithelial tissue is fibroblast-free tissue.

For example, the use as a promotor of tissue re-epithelialization as used herein according to some examples and/or aspects refers to the use as a medicament and/or drug and/or agent defined by its function as a promotor of tissue re-epithelialization.

For example, the use as a promotor of tissue re-epithelialization or the use as a medicament and/or drug and/or agent defined by its function as a promotor of tissue re-epithelialization may comprise any one or combination of the following: a use in treatment of lesions, a use in treatment of acute and/or chronic wounds, a use in treatment of burns, a use in dermatologic procedures, a use in treatment of chronic ulcers, a use in treatment of corneal abrasions, a use in post-surgical recovery, a use in treatment of inflammatory skin diseases, a use in treatment and/or prevention of acne scars, a use in gingival grafting and/or a use in tissue-engineering of skin grafts.

An advantage could be that galactose and/or fucose monosaccharides, in particular in a composition comprising both monosaccharides, may exert healing properties of lesions and/or promotion of re-epithelialization. It may be known that polysaccharides may also exert said properties, but for a person skilled in the art it may be known that epithelial tissue may not offer the environment to break down polysaccharides into the respective enzymes. For that, specific enzymes such as e.g., amylase, glucosidase, and others would need to be present, which may be missing at the target area, e.g., epithelial tissue, in particular epithelial tissue of the human skin. Thus, for the person skilled in the art who may be aware of the fact that polysaccharides exert healing properties of lesions and/or promotion of re-epithelialization, it may not be obvious to conclude that monosaccharides of galactose and fucose may also be used for healing properties of lesions and/or promotion of re-epithelialization.

Another advantage could be that the combination of galactose and fucose has the potential to accelerate the lesion-healing process by promoting re-epithelialization. This could lead to faster closure of lesions, potentially reducing the risk of infection and complications associated with prolonged healing times. By e.g., enhancing the body's natural healing processes, patients may experience reduced scarring and improved cosmetic outcomes. This may be particularly important in visible areas where scars may have a significant psychological impact.

Another advantage could be that since galactose and fucose may be naturally occurring monosaccharides, their use may be perceived as safer and more biocompatible compared to other non-naturally occuring agents. This could lead to fewer side effects, a lower risk of adverse reactions, and a potential increase in compliance by a patient to a therapy or use that e.g., comprises re-epithelialization.

Another advantage could be that beyond promoting re-epithelialization, galactose and fucose may also have roles in modulating the immune response and inflammation, which may be crucial aspects of the wound healing process. Their inclusion in a therapeutic composition could provide a multifaceted approach to wound care. Furthermore, another advantage could be that by potentially speeding up e.g., the healing process and reducing the need for prolonged treatments or management of complications, promoters of tissue re-epithelialization may contribute to lower overall healthcare costs.

Another advantage could be that given the potential chemical properties of the composition, galactose and fucose monosaccharides could be easily incorporated into various formulations, including but not limited to creams, gels, sprays, or dressings, making the treatment e.g., adaptable to different types of use cases and user preferences. Some of the substances described in the state of the art for use as promotors of tissue re-epithelialization and/or for use in treating lesions are polysaccharides in a hydrogel form. Those substances may have disadvantages in terms of shelf life, as a hydrogel is a colonization ground for germs and/or fungi. In addition, the polysaccharides may absolutely be required in a hydrogel formulation, as polysaccharides forms may ensure the structural integrity of the hydrogel, which may not be the case for monosaccharides. The prior art may have thus seen the polysaccharides as important structuring agents for wound healing gels and may have not acknowledged that some monosaccharides or combinations of monosaccharides could have their own therapeutic effect.

Thus, the state of the art may have assumed that polysaccharides may be absolutely needed for the structural stability of gels and because the polysaccharides may have a therapeutic effect in promoting tissue re-epithelialization and/or in treating lesions. The skilled person therefore has no reason to believe that monosaccharides may be useful because no structural stabilization of gels may be possible using the claimed monosaccharides and it may be known that the chemical effect of monosaccharides as compared to polysaccharides may also be fundamentally different. Thus, the combination of galactose and fucose, which in the state of the art may only be known as building blocks structurally comprised and chemically bonded by and within polysaccharides, may also have a promotional effect on tissue re-epithelialization and/or may have healing tendencies for lesions, which cannot be anticipated by the skilled person with regards to the prior art.

An advantage of using monosaccharides may be that monosaccharides may be single sugar units and may have simpler structures compared to polysaccharides, which may be composed of multiple sugar units. This simplicity could lead to more predictable and uniform chemical and physical behaviors in pharmaceutical formulations.

Another advantage of using monosaccharides could be increased solubility, as monosaccharides may generally have higher solubility in water and other solvents compared to polysaccharides. This could make them easier to work with in liquid formulations, such as solutions and creams, where complete dissolution of the active ingredients may be crucial for effectiveness and stability.

Another advantage of using monosaccharides may be that monosaccharides could be absorbed and utilized by the body more rapidly than polysaccharides, which may have to be broken down into simpler sugars before absorption by the body or before passing through biomembranes, e.g., by transmembrane transporters. At various locations in the body, the enzymes breaking down polysaccharides into their respective monosaccharides may be missing, which could hinder or prevent the process of breaking down polysaccharides into their respective monosaccharides. This could make monosaccharides potentially more effective for quicker modes of action, e.g., when being absorbed by the body or before passing through biomembranes.

Another advantage of using monosaccharides may be that monosaccharides may offer greater stability in formulations than polysaccharides, which could be susceptible to depolymerization or other degradation processes. This could be crucial for the shelf-life and efficacy of pharmaceutical products.

Another advantage of using monosaccharides may be that e.g., due to their simpler structure and higher solubility, monosaccharides may demonstrate improved bioavailability in comparison to polysaccharides. This could mean that a greater portion of the active ingredient may be available to exert its therapeutic effect.

Another advantage of using monosaccharides in formulations for oral use, such as inside an oral cavity or on a gingiva, may be that monosaccharides may be sweet and could be used to improve the palatability of oral medications. This may be especially useful in e.g., pediatric and geriatric formulations, where taste could significantly impact patient compliance. Furthermore, solutions of monosaccharides may be less viscous than those containing high concentrations of polysaccharides, which could make them easier to process, formulate, and administer, especially in ophthalmic formulations and formulations to be administered as a solution. Furthermore, the viscosity could be controlled using additional ingredients.

Another advantage could be that monosaccharides may also be chemically modified to participate in controlled-release mechanisms, e.g., offering more precise control over drug release rates.

In another aspect, a composition comprising galactose in monosaccharide form for use as a promotor of tissue re-epithelialization is disclosed. The use as a promotor of tissue re-epithelialization comprises the administration of galactose and fucose in monosaccharide form according to aspects or examples of the invention.

In another aspect, a composition comprising fucose in monosaccharide form for use as a promotor of tissue re-epithelialization is disclosed. The use as a promotor of tissue re-epithelialization comprises the administration of galactose and fucose in monosaccharide form according to aspects or examples of the invention.

For example, the use as a promotor of tissue re-epithelialization comprises an increase in epithelial cell viability.

An advantage could be that by increasing epithelial cell viability, the composition may directly contribute to the enhancement of the re-epithelialization process. This may be critical for e.g., the rapid closure of wounds or for fostering processes where re-epithelialization is desirable, potentially reducing the overall healing time and minimizing the risk of infections or complications.

For example, the use as a promotor of tissue re-epithelialization increases the cell viability above 105% the latest after 72 hours, in particular the latest after 42 hours, from a base value of 100%. The cell viability may be determined via measuring optical density using an MTT assay at 570 nm wavelength.

For example, galactose is D-galactose and fucose is L-fucose.

For example, the tissue is fibroblast-free epithelial tissue, in particular epithelial tissue located in any one or a combination of the following: a skin, in particular fibroblast-free epidermal tissue of a skin, a respiratory tract, an oral cavity, a nasal mucosa, an intestine, a vagina, a cornea, an ear, a urinary tract, a peritoneum, a pleura.

For example, the composition is applied topically, in particular to a skin, to an eye, to an oral cavity, nasally, vaginally, to an intestine, to a respiratory tract, to a urinary tract, peritoneally, to a pleural cavity, to an auditory tract.

For example, the composition is administered as a powder, as a patch, as an ointment dressing, as eye drops, as a mouthwash, as a nasal spray, as a suppository, as an inhalable aerosol, as a catheter coating, as ear drops, as a surgical irrigation solution.

One advantage could be that the composition may be used in a wide array of clinical scenarios, from common conditions like e.g., cuts, burns, and abrasions to more complex clinical needs such as e.g., surgical recovery and the management of chronic diseases affecting tissue, in particular epithelial tissues.

Another advantage could be that the composition may be applied in various formulations and delivery methods suited to the targeted tissue, in particular epithelial tissue, whether it be e.g., topical applications for skin and mucosa, inhalation for respiratory tract, or specialized delivery systems for internal tissues. The composition may offer tailored solutions for specific conditions, such as for example: promoting corneal health after abrasions, aiding in the healing of surgical incisions in the oral cavity, or enhancing recovery in the genital tract after childbirth or surgery.

For example, the relative concentration of galactose to fucose is in the range of 1:9 to 9:1, in particular 1:3 to 3:1, in particular 1:1,5 to 1,5:1, in particular 1:1.

It may be advantageous to combine both monosaccharides in a composition instead of using only fucose or galactose, as their combined effect in treating lesions and/or in promoting re-epithelialization of tissue may be higher than using only one of the monosaccharides alone. The applicant has observed that the monosaccharides work particularly well in combination, as opposed to the individual monosaccharides. This could result in more efficient treatment of lesions and/or more efficient promotion of re-epithelialization of tissue, as each monosaccharide may enhance the other's effectiveness or target different aspects of the healing process. Since galactose and fucose may foster treating of lesions and/or promotion of re-epithelialization of tissue through different pathways, using both could activate multiple mechanisms of action simultaneously. This multi-pathway approach can address various facets of treating lesions and/or promoting of re-epithelialization of tissue at different points in the different pathways.

For example, having the ability to adjust the ratio of galactose to fucose allows for customization of the treatment based on specific lesion/tissue characteristics or healing stages, potentially offering flexibility in clinical application. Hence, the proposed range of relative concentration of galactose to fucose may offer flexibility in the treatment/application approach while simultaneously having e.g., just enough of each monosaccharide to be available for the specific pathway/mode of action in promoting re-epithelialization and/or in treating lesions.

For example, a mode of action of galactose for promoting tissue re-epithelialization is a stimulation of cell proliferation via an activation of a PI3K/AKT/mTOR pathway due to metabolization of galactose within a Leloir pathway.

For example, a mode of action of fucose for promoting tissue re-epithelialization is a stimulation of cell proliferation via a higher access to fucose monosaccharide molecules as an essential building block of cells.

In another aspect, a dressing, bandage or plaster comprising the composition of any of the previous aspects or examples for use as a promotor of tissue re-epithelialization is disclosed, which can be directly applied to a lesion, a wound, a skin or a tissue for re-epithelialization.

This could be beneficial as a targeted delivery of the active ingredients by the dressing, the bandage or the plaster may be ensured. This localized approach may maximize the concentration of beneficial substances at the site of injury, potentially leading to more efficient and faster healing. Furthermore, dressings, bandages, or plasters designed for direct application may be easy to use, e.g., allowing for self-application by patients or caregivers. This could improve patient compliance with the treatment regimen and may ensure that the therapeutic benefits of the composition are realized.

Besides e.g., delivering therapeutic agents, the dressing, bandage, or plaster may also protect the wound from external contaminants and physical trauma. This protective barrier could prevent infection and further injury, both of which are crucial for optimal healing.

Another advantage could be that the composition comprised by the dressing, bandage, or plaster could be formulated for sustained or altered release of the active ingredients. This controlled release may ensure that therapeutic levels of the substances may be maintained over a prolonged period, e.g., reducing the need for frequent dressing changes.

In another aspect, a liquid or semi-solid composition, in particular a solution, a gel, a cream or an ointment, comprising the composition of any of the previous aspects or examples for use as a promotor of tissue re-epithelialization is disclosed, which can be directly applied to a lesion, a wound, a skin or a tissue for re-epithelialization.

One advantage could be an increased versatility in application, as the flexibility of form, ranging from liquid to semi-solid (including solutions, gels, creams, and ointments), may allow for tailored application depending on the wound type, location, and patient preference. This versatility may ensure broader usability across different scenarios, from minor abrasions to more substantial skin lesions. Furthermore, liquid and semi-solid formulations could enhance the penetration of active ingredients into the skin. Their formulation may allow them to deliver therapeutic agents directly to the affected area, for example ensuring that the agents are absorbed efficiently and begin promoting healing and re-epithelialization more effectively than some solid forms might.

Furthermore, solutions, gels, creams, and ointments may generally be easy to apply and may be spread smoothly over the skin, including on tender or painful areas. This ease of use may enhance patient compliance with the treatment regimen, while e.g., the soothing nature of some formulations can provide immediate relief from discomfort.

In another aspect, a composition comprising galactose and fucose in monosaccharide form for use in treating lesions in an individual is disclosed.

For example, the lesions are lesions in epithelial tissue. For example, the lesions are lesions in epithelial tissue of a skin. For example, the lesions are lesions in epidermal tissue. For example, epithelial tissue of a skin comprises epidermal cells. For example, the lesions are lesions in a tissue of a vertebrate, in particular of a mammal, in particular of a human. For example, the lesions are lesions in fibroblast-free tissue. For example, the lesions are lesions in tissue in an epidermis, in an epithelial tissue in the central cornea, in an engineered tissue.

In another aspect, a composition comprising galactose in monosaccharide form for use in treating lesions in an individual is disclosed. The use in treating lesions in an individual comprises the administration of galactose and fucose in monosaccharide form according to aspects or examples of the invention.

In another aspect, a composition comprising fucose in monosaccharide form for use in treating lesions in an individual is disclosed. The use in treating lesions in an individual comprises the administration of galactose and fucose in monosaccharide form according to aspects or examples of the invention.

For example, the lesions occur due to any one or a combination of the following: circulatory disorders of the arteries and/or veins, diabetes mellitus, malnutrition, infections, injuries, postoperative wound healing disorders or a combination and wherein in particular the wounds are pressure ulcers, burn wounds, abrasive wounds, diabetic foot ulcers, venous ulcers, arterial insufficiency ulcers.

For example, the use in treating lesions in an individual comprises an increase in epithelial cell viability.

For example, the use in treating lesions in an individual increases the cell viability above 105% the latest after 72 hours, in particular the latest after 42 hours, from a base value of 100% as determined, for example, via measuring optical density using an MTT assay at 570 nm wavelength.

For example, the lesions are located in any one or a combination of the following: a skin, in particular fibroblast-free epidermal tissue of a skin, a respiratory tract, an oral cavity, a nasal mucosa, an intestine, a vagina, a cornea, an ear, a urinary tract, a peritoneum, a pleura.

For example, a mode of action of galactose for treating lesions is a stimulation of cell proliferation via an activation of a PI3K/AKT/mTOR pathway due to metabolization of galactose within a Leloir pathway.

For example, a mode of action of fucose for treating lesions is a stimulation of cell proliferation via a higher access to fucose monosaccharide molecules as an essential building block of cells.

In another aspect, a dressing, bandage or plaster comprising the composition of any of the previous aspects or examples for use in treating lesions in an individual is disclosed, which can be directly applied to a lesion.

In another aspect, a liquid or semi-solid composition, in particular a solution, a gel, a cream or an ointment, comprising the composition of any of the previous aspects or examples for use in treating lesions in an individual is disclosed, which can be directly applied to a lesion.

In another aspect, a composition comprising galactose and fucose in monosaccharide form for use in treating lesions in an individual is disclosed.

It is understood that the composition comprising galactose and fucose in monosaccharide form for use in treating lesions in an individual may be combined with aspects and/or examples of the composition comprising galactose and fucose in monosaccharide form for use as a promotor of tissue re-epithelialization. Furthermore, it is understood that the advantages of the composition comprising galactose and fucose in monosaccharide form for use as a promotor of tissue re-epithelialization are also applicable to the composition comprising galactose and fucose in monosaccharide form for use in treating lesions in an individual.

For example, the composition comprising galactose and fucose in monosaccharide form could be viewed as a composition for use as a promotor of tissue re-epithelialization (use case a) and/or as a composition for use in treating lesions in an individual (use case b). The aspects and/or examples and/or advantages of use case a and use case b may be combined as long as the combined examples and/or aspects are not mutually exclusive. For example, the lesion is a lesion in a vertebrate, in particular in a mammal, in particular in a human. For example, the lesion is comprised by fibroblast-free tissue. For example, the lesion is comprised by the epidermis.

For example, a lesion comprises any internal or external abnormal tissue or damage in a body. For example, a lesion results from an internal or external disease, injury, or a congenital condition in a body. For example, a lesion comprises internal or external cuts, bruises, burns, tumors, ulcers, and/or infections. For example, internal or external wounds, which are injuries that break the skin or other body tissues, may be a specific type of lesion characterized by disruption of the normal structure and function of the affected tissue. For example, all wounds may be lesions, but not all lesions may be wounds, as the term "lesion" may also cover abnormalities that may not necessarily involve injury or breakage of tissues. For example, the lesion is a wound. For example, the wound is a chronic wound. For example, the wound is an internal and/or an external wound. For example, the internal and/or external wound is inside or outside the human body.

In another aspect, a method of promoting tissue re-epithelialization in a patient in need thereof is disclosed. The method comprises: Administering to the patient a therapeutically effective amount of a combination of sugars selected from the group consisting of galactose and fucose.

In another aspect, a pharmaceutical composition for promoting tissue re-epithelialization is disclosed. The pharmaceutical composition comprises: A therapeutically effective amount of a combination of sugars selected from the group consisting of galactose and fucose; and optionally: A pharmaceutically acceptable carrier.

In another aspect, a use of a combination of sugars selected from the group consisting of galactose and fucose in the manufacture of a medicament for promoting tissue re-epithelialization is disclosed.

In another aspect, a process for preparing a pharmaceutical composition for promoting tissue re-epithelialization is disclosed. The process comprises: Combining a therapeutically effective amount of galactose and fucose and optionally of a pharmaceutically acceptable carrier to form the pharmaceutical composition.

It is understood that aspects and/or examples of the composition comprising galactose and fucose in monosaccharide form of use case b may be combined with the method of promoting tissue re-epithelialization and/or with the pharmaceutical composition for promoting tissue re-epithelialization and/or with the use of a combination of sugars for promoting tissue re-epithelialization and/or with the process for preparing a pharmaceutical composition for promoting tissue re-epithelialization, as long as the combined examples and/or aspects are not mutually exclusive.

In another aspect, a method of treating lesions in a patient in need thereof is disclosed. The method comprises: Administering to the patient a therapeutically effective amount of a combination of sugars selected from the group consisting of galactose and fucose.

In another aspect, a pharmaceutical composition for treating lesions is disclosed. The pharmaceutical composition comprises: A therapeutically effective amount of a combination of sugars selected from the group consisting of galactose and fucose; and optionally: A pharmaceutically acceptable carrier.

In another aspect, a use of a combination of sugars selected from the group consisting of galactose and fucose in the manufacture of a medicament for treating lesions is disclosed.

In another aspect, a process for preparing a pharmaceutical composition for treating lesions is disclosed. The process comprises: Combining a therapeutically effective amount of galactose and fucose and optionally of a pharmaceutically acceptable carrier to form the pharmaceutical composition.

It is understood that aspects and/or examples of the composition comprising galactose and fucose in monosaccharide form of use case a may be combined with the method of treating lesions and/or with the pharmaceutical compositions for treating lesions and/or with the use of a combination of sugars for treating lesions and/or with the process for preparing a pharmaceutical composition for treating lesions, as long as the combined examples and/or aspects are not mutually exclusive.

It is understood that one or more of the aforementioned examples and/or aspects may be combined as long as the combined examples and/or aspects are not mutually exclusive.

### BRIEF DESCRIPTION OF THE DRAWINGS

In the following, examples are described in greater detail making reference to the drawings in which:
Fig. 1 shows a sectional view of the human skin.
Fig. 2 shows a sectional view of the human skin, comprising a lesion.
Fig. 3 shows a results table of an in vitro test of a composition comprising galactose and fucose on in vitro skin epithelial cells of human origin.
Fig. 4 shows a method of promoting tissue re-epithelialization.
Fig. 5 shows a method of treating lesions.
Fig. 6 shows the structural formulas of D-galactose and L-fucose in the fisher projection.

### DETAILED DESCRIPTION

The following paragraphs describe example implementations of the invention.

Without being bound to theory, the present invention is based on the observation that the use of monosaccharides galactose and fucose supports cellular processes critical to re-epithelialization by increasing the proliferation of human epithelial cells, e.g., human skin epithelial cells, e.g., epidermal cells, which do not comprise fibroblasts, i.e., are fibroblast-free.

Without wishing to be bound by theory, the application of a composition comprising galactose onto a region comprising human epithelial cells leads to more substrate availability of galactose to be metabolized via the Leloir pathway, which in turn leads to activation of the PI3K/AKT/mTOR (PAM) signaling pathway. As outlined in Glaviano A, Foo ASC, Lam HY, Yap KCH, Jacot W, Jones RH, Eng H, Nair MG, Makvandi P, Geoerger B, Kulke MH, Baird RD, Prabhu JS, Carbone D, Pecoraro C, Teh DBL, Sethi G, Cavalieri V, Lin KH, Javidi-Sharifi NR, Toska E, Davids MS, Brown JR, Diana P, Stebbing J, Fruman DA, Kumar AP. PI3K/AKT/mTOR signaling transduction pathway and targeted therapies in cancer. Mol Cancer. 2023 Aug 18;22(1):138. Doi: 10.1186/s12943-023-01827-6. PMID: 37596643; PMCID: PMC10436543, the PAM pathway is comprised by the signal transduction network found in eukaryotic cells, which is known for its high conservation and plays a pivotal role in promoting cell survival, growth, and progression through the cell cycle. This network involves the transmission of signals from growth factors to transcription factors within the PAM axis, a process that is intricately controlled through numerous interactions with various other signaling pathways. Furthermore, when there is a disruption in this signal transduction process, it can lead to an increased risk of developing cancer. A higher availability of galactose fosters re-epithelialization and/or treatment of lesions via promoting cell survival, growth, and progression through the activation of the PAM pathway. In other words, an increased access to galactose activates the PAM pathway, which in turn stimulates cell proliferation.

The Leloir pathway is a biochemical pathway for the metabolism of galactose. The Leloir pathway converts galactose into UDP-galactose, which is then converted into glucose-1-phosphate. Galactose is first phosphorylated to galactose-1-phosphate by the enzyme galactokinase. Galactose-1-phosphate is then converted into UDP-galactose by the enzyme galactose-1-phosphate uridylyltransferase, using UDP-glucose as a co-substrate. UDP-galactose is converted into UDP-glucose by the enzyme UDP-galactose 4-epimerase. Finally, UDP-glucose can enter various metabolic pathways, including being converted into glucose-1-phosphate.

Furthermore, without wishing to be bound by theory, fucose is an essential building block of human cells and a higher access to this monosaccharide contributes to cell growth. Referencing to Yao H, Shi H, Jiang C, Fan M, Zhang Y, Qian W, Lin R. L-Fucose promotes enteric nervous system regeneration in type 1 diabetic mice by inhibiting SMAD2 signaling pathway in enteric neural precursor cells. Cell Commun Signal. 2023 Oct 5;21(1):273. Doi: 10.1186/s12964-023-01311-0. PMID: 37798789; PMCID: PMC10552466, fucose has been shown to be involved in a number of cellular pathways and significantly contributes to cell differentiation and regeneration which are also important for re-epithelialization and/or treatment of lesions.

The following paragraph outlines examples as well as example in vitro data of the invention.

In the following, similar elements are denoted by the same reference numerals.

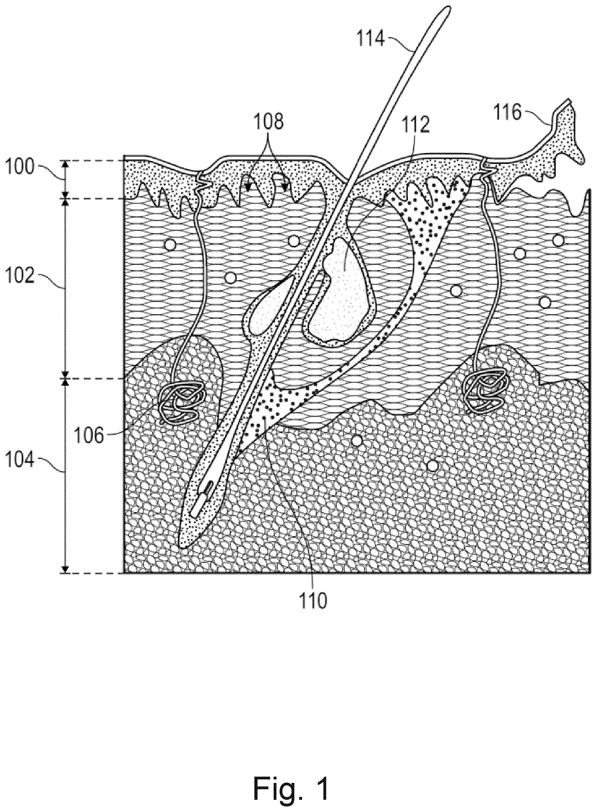

Figure 1 shows a sectional view of the human skin.

Figure 1 depicts an epidermis 100, a dermis 102, a hypodermis 104, a sweat gland 106, a dermal papillae 108, an arrector pili muscle 110, a sebaceous gland 112, a hair 114 and an outer layer of keratin 116.

In figure 1, the uppermost layer comprises the covering epithelia, the keratinized squamous epithelium/the outer layer of keratin 116 of the skin. Right below the outer layer of keratin 116, the epidermis 100 is located, comprising fibroblast-free epidermal cells, which are a specific type of epithelial cells. The layer below the epidermis 100 comprises the dermis 102, wherein the main cell population forming the dermis 102 are fibroblasts.

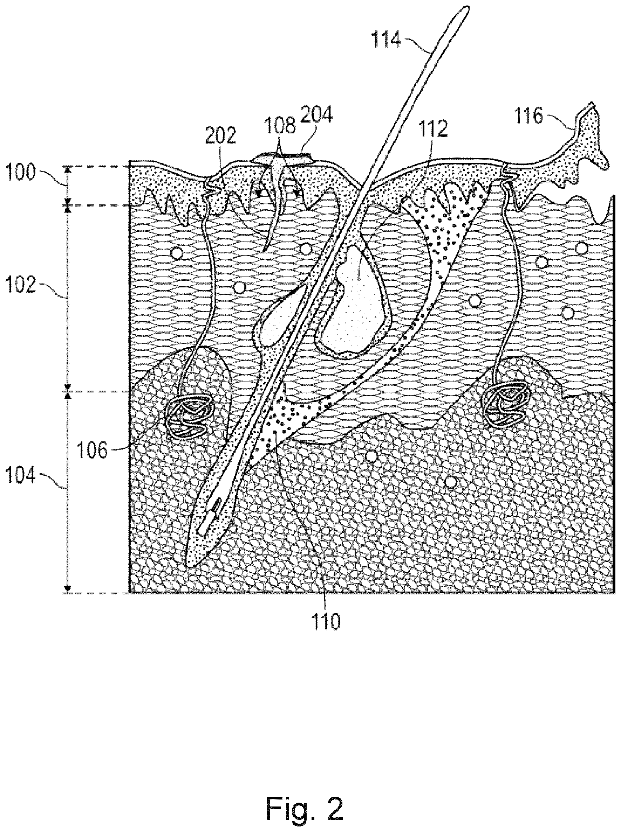

Figure 2 shows a sectional view of the human skin, comprising a lesion 202.

In the example of figure 2, the lesion 202 is an external chronic wound, but could also be any other internal or external type of lesion according to the aforementioned examples. Figure 2 depicts the lesion 202 extending through the epidermis 100 into the dermis 102. Although not shown, the lesion could also only affect the epidermis 100, without affecting the dermis 102. Furthermore, figure 2 depicts a test item 204 applied on top of the lesion 202. In the example of figure 2, the test item 204 is D-galactose and L-fucose monosaccharides as a powder mixture with a relative ratio of 1:1 in weight, comprised by a cream. In the example, the test item 204 is dissolved in the water component of the cream, so the individual particles of the powder mixture are dissolved and not visible in figure 2. However, the test item 204 could also be applied as a powder directly, or be comprised by any other pharmaceutically appropriate form to be applied to the human skin according to aforementioned examples. In lesions, such as chronic wounds, both epidermis 100 and dermis 102 may be affected. In another example, only the epidermis 100 may be affected. Healing of lesions may start in the dermis 102, where fibroblasts could proliferate and new blood vessels and inflammatory cells may evade from the bloodstream, e.g., forming the granulation tissue as a special form of regenerative tissue. If the connective tissue layer may have healed, the epithelium of the epidermis 100 could overgrow from the margin of the wound to e.g., finally close the wound with an epithelial layer. Once this may have been achieved, the wound may have healed.

In the example of figure 2, the composition comprising the combination of galactose and fucose promotes the proliferation of human skin epithelial cells, thus promoting re-epithelialization of the epithelial cells and/or treating the lesion 202, resulting in, for example, stimulation of epidermal growth and/or growth of tissue, enhanced lesion healing and/or wound healing, improving skin barrier function, reducing inflammation, and/or enhancing hydration.

In the example (not shown) where the lesion 202 only affects the epidermis 100 but not the dermis 102, the test item 204 would still foster re-epithelialization of tissue and/or healing of lesions, as the test item 204 is also effective in fibroblast-free tissue.

It is understood that in the example of figure 1 and 2 comprising human tissue such as e.g., the human skin and the epidermis, no capacity to break down polysaccharides into their respective monosaccharides may be offered. The epidermis primarily serves as a barrier to protect the body from external environmental factors, including pathogens, chemicals, and physical harm, rather than hosting enzymes for polysaccharide digestion. Most enzymatic breakdown of polysaccharides occurs within the digestive system, where enzymes such as amylase, glucosidase, and others efficiently degrade dietary polysaccharides into monosaccharides for absorption and use by the body. The human skin, e.g., the epidermis, lacks the specific enzymatic machinery designed for the extensive breakdown of polysaccharides into monosaccharides as seen in other parts of the body. Thus, applying a composition comprising polysaccharides, that may comprise fucose and/or galactose, may be of no use to achieve features of the invention, as polysaccharides may not be broken down into the respective monosaccharides. In light of the claimed features, the smaller monosaccharide molecules may e.g., be more readily utilized or absorbed by the skin without the need for extensive enzymatic breakdown.

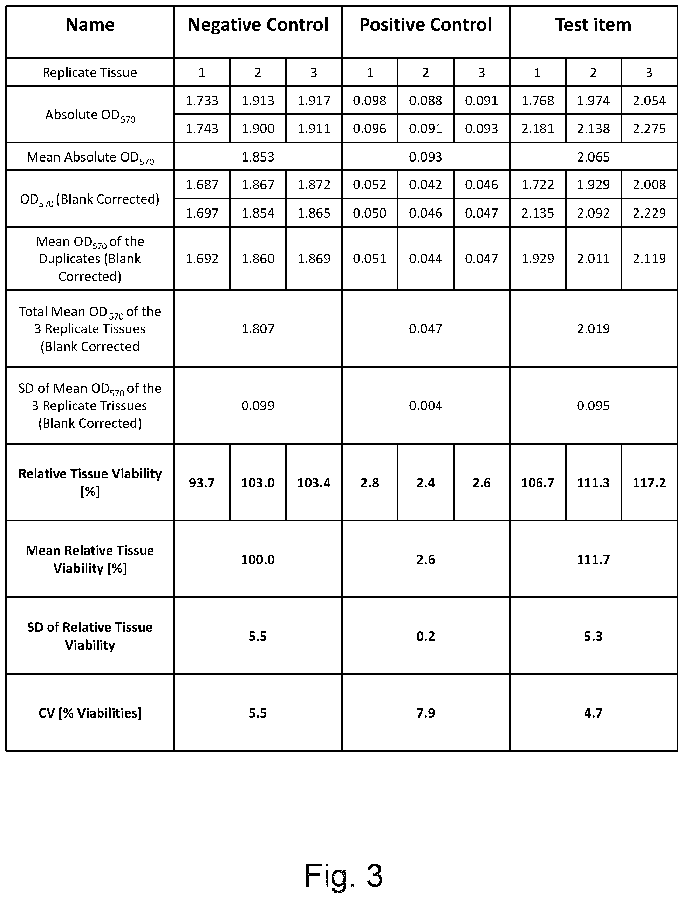

Figure 3 shows a results table of an in vitro test of a composition comprising galactose and fucose on in vitro skin epithelial cells of human origin.

In the example, a test comprising the EpiDerm^{™} reconstructed human epidermis model (MatTek) is used, which comprises normal human epidermal keratinocytes (NHEK) and therefore represents in vitro the target organ of the species of interest (epidermis, human). It closely mimics the biochemical and physiological properties of e.g., the upper parts of the human skin, i.e., the epidermis and its epithelial cells, which do not comprise fibroblasts. Epidermal cells are a specific type of epithelial cells. Epidermal cells are fibroblast-free.

It is understood that the EpiDerm^{™} reconstructed human epidermis model imitates normal human epidermal cells in a way that it at least does not contain fibroblasts. The proposed mode of action for D-galactose as described e.g. in Kössi J, Peltonen J, Ekfors T, Niinikoski J, Laato M. Effects of hexose sugars: glucose, fructose, galactose and mannose on wound healing in the rat. Eur Surg Res. 1999;31(1):74-82. Doi: 10.1159/000008623 may be an increase in granulation tissue production after the administration of galactose using a rat model, wherein the granulation tissue comprises fibroblasts. As the EpiDerm^{™} model does not comprise fibroblasts and/or granulation tissue, the mode of action of galactose of the claimed composition comprising both galactose and fucose has to differ from the mode of action as described in Kössi et. al (1999) for galactose.

For testing, D-galactose (Chemical Abstracts Service, CAS No. 59-23-4) and L-fucose (CAS No. 2438-80-4) monosaccharides are used, and the term "test item" refers to a solid composition comprising D-galactose and L-fucose monosaccharides as a powder mixture with a relative ratio of 1:1 in weight. The purity of the monosaccharides was 99.8% for D-galactose and 100% for L-fucose.

Firstly, 25 µL of sterile Dulbecco's phosphate buffered saline, DPBS (Cat. No. 14040-091), was applied to the epidermal surface of the EpiDerm^{™} tissue in order to improve the contact between the test item as a powder mixture and the epidermis model. Afterwards, 25 mg (^{~}42 mg/cm²) of the test item was applied directly atop the EpiDerm^{™} tissue using an application spoon avoiding compression of the test item. The test item formed a suspension with the DPBS. A nylon mesh was placed on top of the mixture to evenly spread the mixture and to promote equal contact with the skin. The compatibility of the test item with the nylon mesh was checked in a pre-test by applying 25 mg of the test item together with 25 µL PBS to a nylon mesh and placing it on a slide. After 60 min the mesh was examined microscopically. If the test item mixture did not interact with the mesh, the mesh did not interact with the test item and was placed on top of the mixture after its application to the EpiDerm^{™} tissue.

Two control groups (negative control group, positive control group) were set up in parallel to the test item group (test item group) in order to confirm the validity of the test, with DPBS being the negative control and 5% sodium dodecyl sulfate (CAS No. 151-21-3) being the positive control. Furthermore, 30 µL of the ngeative control in the negative control group and 30 µL of the positive control in the positive control group were used and, as outlined above, 25 mg of the test item + 25 µL DPBS were used in the test item group. The test was performed on a total of 3 EpiDerm^{™} tissues per dose group.

The test was carried out with the reconstituted three-dimensional human skin model EpiDerm^{™}. This skin model comprises normal human epidermal keratinocytes (NHEK) which have been cultured to form a multi-layered, highly differentiated model of the human epidermis. The NHEK are cultured on chemically modified, collagen-coated cell culture inserts. The EpiDerm^{™} epidermis model exhibits in vivo-like morphological and growth characteristics which are uniform and highly reproducible. It consists of organized basal, spinous and granular layers and a multi-layered stratum corneum analogous to patterns found in vivo. The EpiDerm^{™} tissues were provided as a sealed 24-well plate containing e.g., 24 reconstructed epidermis units (area: 0.6 cm²), wherein each reconstructed epidermis is attached to a cell culture insert and maintained on nutritive agar for transport.

Furthermore, 3-(4,5-dimethylthiazol-2-yl)-2,5-diphenyltetrazolium bromide, MTT solutions were provided: an MTT stock solution with 5 mg/mL MTT, and an MTT medium/assay medium, wherein the MTT stock solution was diluted 1 + 4 with Dulbecco's Modified Eagle Medium, DMEM-based medium. In addition, isopropanol and distilled water were provided.

The EpiDerm^{™} tissues were inspected visually and transferred into 6-well plates containing 0.9 mL assay medium per well. The surface was dried using a sterile cotton tip and the plates were incubated in a humidified incubator at 37 ± 1 °C, 5.0% CO2 for 60 ± 5 min. Subsequently, the tissues were transferred into new wells containing 0.9 mL pre-warmed assay medium per well and were incubated for 18 - 21 h in a humidified incubator at 37 ± 1 °C, 5.0% CO2.

After this pre-incubation the tissues were treated with each dose group in triplicate, starting with the negative control. Start time was recorded with dosing of the first tissue and occurred sequentially for the other tissues, e.g., in one-minute intervals. After dosing of all tissues, all plates were incubated for 25 ± 1 min under a sterile airflow and for the remaining time of 35 ± 1 min transferred to the incubator. Then the tissues were washed by filling and emptying the inserts 15 times with DPBS using a constant stream about 1.5 cm distance from the tissue surface, this process also occurred sequentially, e.g., in one-minute intervals. Subsequently, the inserts were completely submerged three times in 150 mL DPBS and shaken to remove the rest of the test item. Finally, the inserts were rinsed once from the inside and the outside with sterile DPBS. Excess DPBS was removed by blotting the bottom with blotting paper. The inserts were placed in prepared new 6-well plates containing 0.9 mL pre-warmed fresh assay medium per well and the tissue surface was dried using a sterile cotton tip. The plates were post-incubated at 37 ± 1 °C, 5.0% CO2, humidified to 95%, for 24 ± 2 h. Following this incubation, the tissues were transferred to new wells containing 0.9 mL fresh assay medium and incubated for additional 18 ± 2 h.

After this post-incubation period, the bottom of the inserts were blotted on sterile blotting paper and the inserts were transferred in a prepared 24-well plate containing 300 µL pre-warmed MTT medium. This plate was incubated in the MTT incubation period for 3 h ± 5 min at 37 ± 1 °C, 5.0% CO2, and humidified to 95%.

After the MTT incubation period, the tissues were rinsed three times with DPBS and afterwards placed on blotting paper to dry. The tissues were transferred into 12-well plates and immersed in 2 mL isopropanol, sealed to inhibit evaporation. Extraction was carried out protected from light at room temperature for at least 2 h with gentle shaking on a plate shaker.

Before using the extracts, the plate had been shaken for at least 15 min on a plate shaker and the inserts were pierced with an injection needle. The extract was pipetted up and down 3 times before 2 x 200 µL aliquots per each tissue were transferred into a 96-well plate. The optical density (OD) was measured in a micro plate auto reader (Tecan Infinite M200 Pro, Tecan, Austria) at 570 nm (wavelength accuracy ≤ ± 1.5 nm λ > 315 nm/ ≤ ± 0.8nm λ ≤ 315 nm) using isopropanol as a blank. The optical density (OD) may serve as a measure for the tissue viability in the following results.

Figure 3 shows a table comprising the results of measuring the optical density, OD₅₇₀, of the three groups. In figure 3, blank-corrected mean OD₅₇₀ of the negative control corresponds to 100% absolute tissue viability.

Figure 3 shows that within the test item group, a mean relative tissue viability of 111.7% was achieved, as compared to 100.0% in the negative control group. Our results may be interpreted in that the test item, comprising D-galactose and L-fucose monosaccharides as a powder mixture with a relative ratio of 1:1 in weight, may increase the cell viability of epithelial cells and/or epithelial tissue by 111.7%. Thus, in comparison to the negative control group, the value over 100% could mean that the treated epithelial cells or the treated fibroblast-free tissue is more viable than the (negative) control cells or tissue.

Our results may indicate the epithelial cells are particularly proliferative after the application of the test item. This could be due to the activation of the pathways and/or mode of actions outlined in the respective paragraphs, which e.g., promote epithelial cell survival and growth as a response to the treatment with the test item.

An increase in cell viability, particularly of epithelial cells, may have significant implications for treating lesions and facilitating processes where re-epithelialization may be desired. Re-epithelialization could be a critical phase of wound healing or healing of lesions, e.g., involving the migration and proliferation of epithelial cells to cover a wound or lesion, thereby e.g., restoring the integrity of the skin or mucosal barrier. Re-epithelialization may also be beneficial in use cases outlined in other examples of the invention.

An increased viability of epithelial cells may indicate a greater number of healthy, functional cells available to e.g., initiate a wound or lesion healing process. This could lead to more efficient re-epithelialization, reducing the time it takes for wounds or lesions to heal. The primary function of epithelial tissues may be to act as a barrier against environmental threats, including pathogens and physical or chemical damage. Enhanced cell viability may ensure a more robust regeneration of this barrier, potentially restoring its protective capabilities more rapidly and effectively.

A faster re-epithelialization may also be desirable in other processes, for example in including but not limited to a use in gingival grafting and/or a use in tissue-engineering of skin grafts.

The results of the example in figure 3 may be interpreted in a way that a composition comprising D-galactose and L-fucose monosaccharides as a powder mixture with a relative ratio of 1:1 in weight, which may be comprised by other pharmaceutically appropriate forms, could be beneficial in treating lesions and/or in processes where a promotion of tissue re-epithelialization is desired.

In the example of figure 3, we only show an example process to determine the promotion of tissue re-epithelialization and/or the benefit in treating lesions of the test item, using optical density to measure the cell viability of a tissue assay. Other tests or methods may be used to e.g., show an increase in cell viability or to e.g., show the promoting effects of the composition on tissue re-epithelialization or to e.g., show the possibilitiy for treating lesions using the composition.

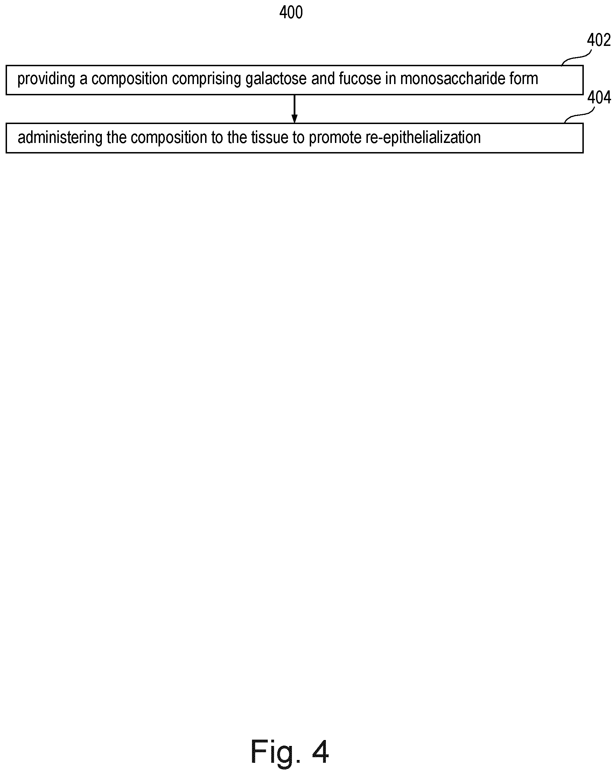

Figure 4 shows a method 400 of promoting tissue re-epithelialization. A composition comprising galactose and fucose in monosaccharide form is provided 402. The composition is administered to the tissue to promote re-epithelialization 404.

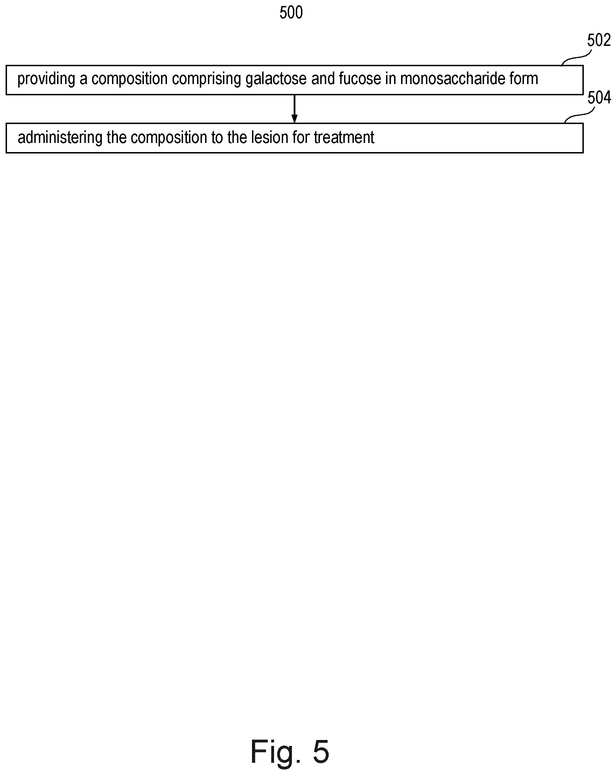

Figure 5 shows a method 500 of treating lesions. A composition comprising galactose and fucose in monosaccharide form is provided 502. The composition is administered to the lesion for treatment 504.

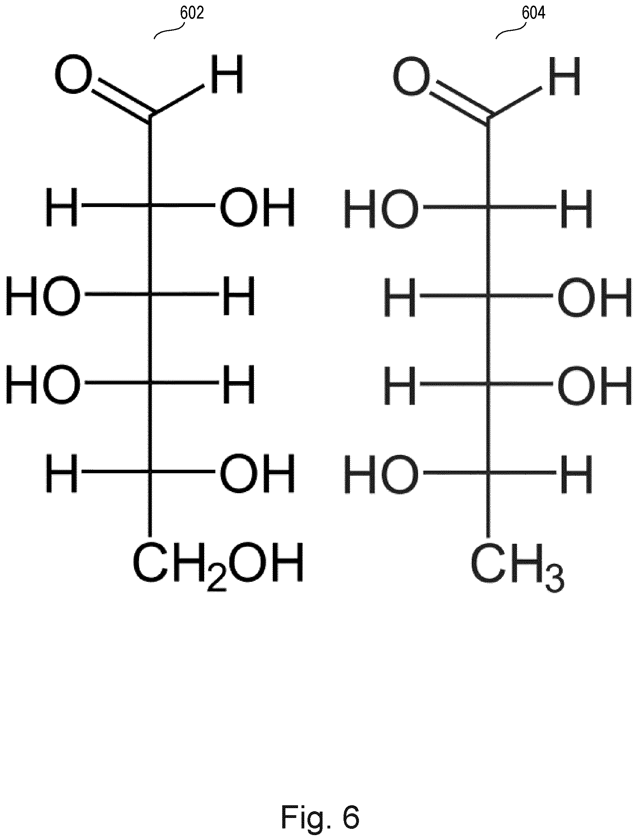

Figure 6 shows the structural formulas of D-galactose 602 and L-fucose 604 in the fisher projection.

While the invention has been illustrated and described in detail in the drawings and foregoing description, such illustration and description are to be considered illustrative or exemplary and not restrictive; the invention is not limited to the disclosed examples.

The term "about" is used to indicate that a value includes the standard deviation of error for the device or method being employed to determine the value. The use of the term "or" in the claims is used to mean "and/or" unless explicitly indicated to refer to alternatives only or the alternatives are mutually exclusive, although the disclosure supports a definition that refers to only alternatives and to "and/or." When used in conjunction with the word "comprising" or other open language in the claims, the words "a" and "an" denote "one or more," unless specifically noted. The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

The invention may also be described by the following set of clauses (clause set 1a).

Clause 1a: A composition comprising galactose and fucose in monosaccharide form for use as a promotor of tissue re-epithelialization.

Clause 2a: A composition comprising galactose in monosaccharide form for use as a promotor of tissue re-epithelialization, wherein the use as a promotor of tissue re-epithelialization comprises the administration of galactose and fucose in monosaccharide form according to clause 1a.

Clause 3a: A composition comprising fucose in monosaccharide form for use as a promotor of tissue re-epithelialization, wherein the use as a promotor of tissue re-epithelialization comprises the administration of galactose and fucose in monosaccharide form according to clause 1a.

Clause 4a: The composition of any of the previous clauses for use as a promotor of tissue re-epithelialization, wherein the use as a promotor of tissue re-epithelialization comprises an increase in epithelial cell viability.

Clause 5a: The composition of clause 4a for use as a promotor of tissue re-epithelialization, wherein the use as a promotor of tissue re-epithelialization increases the cell viability above 105% the latest after 72 hours, in particular the latest after 42 hours, from a base value of 100% as determined, for example, via measuring optical density using an MTT assay at 570 nm wavelength.

Clause 6a: The composition of any of the previous clauses for use as a promotor of tissue re-epithelialization, wherein galactose is D-galactose and fucose is L-fucose.

Clause 7a: The composition of any of the previous clauses for use as a promotor of tissue re-epithelialization, wherein the tissue is fibroblast-free epithelial tissue, in particular epithelial tissue located in any one or a combination of the following: a skin, in particular fibroblast-free epidermal tissue of a skin, a respiratory tract, an oral cavity, a nasal mucosa, an intestine, a vagina, a cornea, an ear, a urinary tract, a peritoneum, a pleura.

Clause 8a: The composition of any of the previous clauses for use as a promotor of tissue re-epithelialization, wherein the composition is applied topically, in particular to a skin, to an eye, to an oral cavity, nasally, vaginally, to an intestine, to a respiratory tract, to a urinary tract, peritoneally, to a pleural cavity, to an auditory tract.

Clause 9a: The composition of any of the previous clauses for use as a promotor of tissue re-epithelialization, wherein the composition is administered as a powder, as a patch, as an ointment dressing, as eye drops, as a mouthwash, as a nasal spray, as a suppository, as an inhalable aerosol, as a catheter coating, as ear drops, as a surgical irrigation solution.

Clause 10a: The composition of any of the previous clauses for use as a promotor of tissue re-epithelialization, wherein the relative concentration of galactose to fucose is in the range of 1:9 to 9:1, in particular 1:3 to 3:1, in particular 1:1,5 to 1,5:1, in particular 1:1.

Clause 11a: The composition of any of the previous clauses for use as a promotor of tissue re-epithelialization, wherein a mode of action of galactose for promoting tissue re-epithelialization is a stimulation of cell proliferation via an activation of a PI3K/AKT/mTOR pathway due to metabolization of galactose within the Leloir pathway.

Clause 12a: The composition of any of the previous clauses for use as a promotor of tissue re-epithelialization, wherein a mode of action of fucose for promoting tissue re-epithelialization is a stimulation of cell proliferation via a higher access to fucose monosaccharide molecules as an essential building block of cells.

Clause 13a: A dressing, bandage or plaster comprising the composition of any of the previous clauses for use as a promotor of tissue re-epithelialization, which can be directly applied to a lesion, a wound, a skin or a tissue for re-epithelialization.

Clause 14a: A liquid or semi-solid composition, in particular a solution, a gel, a cream or an ointment, comprising the composition of any of the previous clauses for use as a promotor of tissue re-epithelialization, which can be directly applied to a lesion, a wound, a skin or a tissue for re-epithelialization.

Furthermore, the invention may also be described by the following set of clauses (clause set 1b).

Clause 1b: A composition comprising galactose and fucose in monosaccharide form for use in treating lesions in an individual.

Clause 2b: A composition comprising galactose in monosaccharide form for use in treating lesions in an individual, wherein the use in treating lesions in an individual comprises the administration of galactose and fucose in monosaccharide form according to clause 1b.

Clause 3b: A composition comprising fucose in monosaccharide form for use in treating lesions in an individual, wherein the use in treating lesions in an individual comprises the administration of galactose and fucose in monosaccharide form according to clause 1b.

Clause 4b: The composition of any of the previous clauses for use in treating lesions in an individual, wherein the lesions occur due to any one of the following: circulatory disorders of the arteries and/or veins, diabetes mellitus, malnutrition, infections, injuries, postoperative wound healing disorders or a combination and wherein in particular the wounds are pressure ulcers, burn wounds, abrasive wounds, diabetic foot ulcers, venous ulcers, arterial insufficiency ulcers or a combination.

Clause 5b: The composition of any of the previous clauses for use in treating lesions in an individual, wherein the use in treating lesions in an individual comprises an increase in epithelial cell viability.

Clause 6b: The composition of clause 5b for use in treating lesions in an individual, wherein the use in treating lesions in an individual increases the cell viability above 105% the latest after 72 hours, in particular the latest after 42 hours, from a base value of 100% as determined, for example, via measuring optical density using an MTT assay at 570 nm wavelength.

Clause 7b: The composition of any of the previous clauses for use in treating lesions in an individual, wherein galactose is D-galactose and fucose is L-fucose.

Clause 8b: The composition of any of the previous clauses for use in treating lesions in an individual, wherein the lesions are located in any one or a combination of the following: a skin, in particular in fibroblast-free epithelial tissue of a skin, a respiratory tract, an oral cavity, a nasal mucosa, an intestine, a vagina, a cornea, an ear, a urinary tract, a peritoneum, a pleura.

Clause 9b: The composition of any of the previous clauses for use in treating lesions in an individual, wherein the composition is applied topically, in particular to a skin, to an eye, to an oral cavity, nasally, vaginally, to an intestine, to a respiratory tract, to a urinary tract, peritoneally, to a pleural cavity, to an auditory tract.

Clause 10b: The composition of any of the previous clauses for use in treating lesions in an individual, wherein the composition is administered as a powder, as a patch, as an ointment dressing, as eye drops, as a mouthwash, as a nasal spray, as a suppository, as an inhalable aerosol, as a catheter coating, as ear drops, as a surgical irrigation solution.

Clause 11b: The composition of any of the previous clauses for use in treating lesions in an individual, wherein the relative concentration of galactose to fucose is in the range of 1:9 to 9:1, in particular 1:3 to 3:1, in particular 1:1,5 to 1,5:1, in particular 1:1.

Clause 12b: The composition of any of the previous clauses for use in treating lesions in an individual, wherein a mode of action of galactose for treating lesions is a stimulation of cell proliferation via an activation of a PI3K/AKT/mTOR pathway due to metabolization of galactose within the Leloir pathway.

Clause 13b: The composition of any of the previous clauses for use in treating lesions in an individual, wherein a mode of action of fucose for treating lesions is a stimulation of cell proliferation via a higher access to fucose monosaccharide molecules as an essential building block of cells.

Clause 14b: A dressing, bandage or plaster comprising the composition of any of the previous clauses for use in treating lesions in an individual, which can be directly applied to a lesion.

Clause 15b: A liquid or semi-solid composition, in particular a solution, a gel, a cream or an ointment, comprising the composition of any of the previous clauses for use in treating lesions in an individual, which can be directly applied to a lesion.

### REFERENCE SIGNS LIST

- 100: epidermis
- 102: dermis
- 104: hypodermis
- 106: sweat gland
- 108: dermal papillae
- 110: arrector pili muscle
- 112: sebaceous gland
- 114: hair
- 116: outer layer of keratin
- 202: lesion
- 204: composition comprising galactose and fucose
- 400: method of promoting tissue re-epithelialization
- 402: providing a composition comprising galactose and fucose in monosaccharide form
- 404: administering the composition to the tissue to promote re-epithelialization
- 500: method of treating lesions
- 502: providing a composition comprising galactose and fucose in monosaccharide form
- 504: administering the composition to the lesion for treatment

## Claims

1. A composition (204) comprising galactose and fucose in monosaccharide form for use as a promotor of tissue re-epithelialization.

2. A composition comprising galactose in monosaccharide form for use as a promotor of tissue re-epithelialization, wherein the use as a promotor of tissue re-epithelialization comprises the administration of galactose and fucose in monosaccharide form according to claim 1.

3. A composition comprising fucose in monosaccharide form for use as a promotor of tissue re-epithelialization, wherein the use as a promotor of tissue re-epithelialization comprises the administration of galactose and fucose in monosaccharide form according to claim 1.

4. The composition of any of the previous claims for use as a promotor of tissue re-epithelialization, wherein the use as a promotor of tissue re-epithelialization comprises an increase in epithelial cell viability.

5. The composition of claim 4 for use as a promotor of tissue re-epithelialization, wherein the use as a promotor of tissue re-epithelialization increases the cell viability above 105% the latest after 72 hours, in particular the latest after 42 hours, from a base value of 100% as determined, for example, via measuring optical density using an MTT assay at 570 nm wavelength.

6. The composition of any of the previous claims for use as a promotor of tissue re-epithelialization, wherein galactose is D-galactose (602) and fucose is L-fucose (604).

7. The composition of any of the previous claims for use as a promotor of tissue re-epithelialization, wherein the tissue is fibroblast-free epithelial tissue, in particular epithelial tissue located in any one or a combination of the following: a skin, in particular fibroblast-free epidermal tissue of a skin, a respiratory tract, an oral cavity, a nasal mucosa, an intestine, a vagina, a cornea, an ear, a urinary tract, a peritoneum, a pleura.

8. The composition of any of the previous claims for use as a promotor of tissue re-epithelialization, wherein the composition is applied topically, in particular to a skin, to an eye, to an oral cavity, nasally, vaginally, to an intestine, to a respiratory tract, to a urinary tract, peritoneally, to a pleural cavity, to an auditory tract.

9. The composition of any of the previous claims for use as a promotor of tissue re-epithelialization, wherein the composition is administered as a powder, as a patch, as an ointment dressing, as eye drops, as a mouthwash, as a nasal spray, as a suppository, as an inhalable aerosol, as a catheter coating, as ear drops, as a surgical irrigation solution.

10. The composition of any of the previous claims for use as a promotor of tissue re-epithelialization, wherein the relative concentration of galactose to fucose is in the range of 1:9 to 9:1, in particular 1:3 to 3:1, in particular 1:1,5 to 1,5:1, in particular 1:1.

11. The composition of any of the previous claims for use as a promotor of tissue re-epithelialization, wherein a mode of action of galactose for promoting tissue re-epithelialization is a stimulation of cell proliferation via an activation of a PI3K/AKT/mTOR pathway due to metabolization of galactose within the Leloir pathway.

12. The composition of any of the previous claims for use as a promotor of tissue re-epithelialization, wherein a mode of action of fucose for promoting tissue re-epithelialization is a stimulation of cell proliferation via a higher access to fucose monosaccharide molecules as an essential building block of cells.

13. A dressing, bandage or plaster comprising the composition of any of the previous claims for use as a promotor of tissue re-epithelialization, which can be directly applied to a lesion, a wound, a skin or a tissue for re-epithelialization.

14. A liquid or semi-solid composition, in particular a solution, a gel, a cream or an ointment, comprising the composition of any of the previous claims for use as a promotor of tissue re-epithelialization, which can be directly applied to a lesion, a wound, a skin or a tissue for re-epithelialization.

15. A composition (204) comprising galactose and fucose in monosaccharide form for use in treating lesions (202) in an individual.
